# EUROPEAN PATENT SPECIFICATION

(11) **EP 2 178 377 B1**
(45) Date of publication and mention of the grant of the patent: **02.05.2012**
(21) Application number: 08775972.6
(22) Date of filing: 16.07.2008
(51) Int. Cl.: A01N 53/00, A01N 57/12, A01N 25/34, A01P 7/04

(54) **METHOD OF COMBATTING HUMAN HEAD LICE**
VERFAHREN ZUR BEKÄMPFUNG MENSCHLICHER KOPFLÄUSE
MÉTHODE DE LUTTE CONTRE LES POUX DE TÊTE

(30) Priority: 16.07.2007 GB 0713790
(43) Date of publication of application: 28.04.2010
(73) Proprietor: Nettforsk AS, 4801 Norway (NO)
(72) Inventor: JOHANNESSEN, Baard, N-4841 Arendal (NO)
(74) Representative: Cockbain, Julian
(86) International application number: PCT/GB2008/002441
(87) International publication number: WO 2009/010754

(56) References cited:
- EP-A- 0 894 435
- FR-A- 2 793 112
- GB-A- 2 343 627
- US-A- 4 882 873
- US-A- 5 782 799
- G U I L L E T P ET AL: "Combined pyrethroid and carbamate 'two-in-one' treated mosquito nets: TM eld ef TM cacy against pyrethroid-resistant Anopheles gambiae and Culex quinquefasciatus" MEDICAL AND VETERINARY ENTOMOLOGY, BLACKWELL SCIENTIFIC PUBL., OXFORD, GB, vol. 15, 1 January 2001 (2001-01-01), pages 105-112, XP007905295 ISSN: 0269-283X

## Description

This invention relates to a pyrethroid or pyrethrin, or alternatively an organophosphate or carbamate, for use in a method of topical treatment of living human subjects to combat multicellular ectoparasites with exoskeletons, especially ectoparasites of the orders *Phthiraptera* (lice), *Acarina* (mites) and *Siphonaptera* (fleas), more especially *Pediculus humanus capitis* (human head lice).

Many humans are infested with such multicellular ectoparasites, eg ticks, fleas, mites, and lice, especially mites and lice, e.g. the head louse (*Pediculus humanus capitis*), body louse (*Pediculus humanus humanus*), the pubic louse (*Pthirus pubis*) and the scabies mite (*Sarcoptes scabiei*). Head lice, in particular, are a common problem for humans, especially school children. Effective control involves rapid and accurate detection, the use of a fine-toothed comb and the application of head lice killing chemicals, pediculicides.

Pediculicides are frequently toxic to other ectoparasites, eg the mites responsible for scabies, and thus elsewhere may also be referred to as insecticides or scabicides, etc.

The pediculicides currently available generally fall into three classes: organophosphates (eg malathion), carbamates (eg carbaryl), and pyrethroids (eg permethrin).

These pediculicides however have toxic effects. Concerns have long been expressed about organophosphate toxicity in particular, for example in relation to farm workers. Organophosphate poisoning does not require ingestion - cutaneous absorption can lead to signs of poisoning. Symptoms of organophosphate poisoning may include excessive excessive salivation, sweating, rhinorrhea, muscle twitching, weakness, tremor, incoordination, headache, dizziness, nausea, vomiting, abdominal cramps, diarrhoea, respiratory depression, wheezing, blurred vision and more. Carbamates can cause adverse reactions such as sweating, vision blurring, incoordination and convulsions. Pyrethroids similarly can cause adverse reactions even on dermal exposure, such as excitory neurotoxicity, altered dopamine uptake, and dermatitis.

Since head lice infestation is a particular problem for school children, there is thus a need for a head lice treatment with reduced exposure to pyrethroids and organophosphates. Coadministration of both a pyrethroid and an organophosphate has been proposed by Mazars in FR-A-2793112 which describes a device which produces an aerosol simultaneously from a first solution containing an organophosphate (eg malathion) and a second solution containing a pyrethroid (eg permethrin). GB 2343627 discloses dsequential use of various pediculicides, which are typically administered via an article such as a hair net that is impregnated with the pediculicide. We have found however that dermal exposure to these pediculicides may be reduced without compromising efficacy by staggered application of an organophosphate or carbamate and of a pyrethroid, in that order. Thus, the staggered administration according to the invention is more concerned with reducing exposure of the human subject to potentially toxic chemicals than with overcoming ectoparasite resistance to pediculicides.

Viewed from one aspect the invention provides a pyrethroid or pyrethrin pediculicide for use in a method of treatment of a human subject to combat infestation by multicellular ectoparasites with exoskeletons, in particular head lice, which method comprises topically applying to said subject a first and a second pediculicide, said first pediculicide being a carbamate or organophosphate pediculicide and said second pediculicide being a pyrethroid or pyrethrin pediculicide, characterized in that said second pediculicide is applied between 15 minutes and 12 hours after the application of said first pediculicide. Viewed from another aspect the invention provides an organophosphate or carbamate pediculicide for use in a method of treatment of a human subject to combat infestation by multicellular ectoparasites with exoskeletons, in particular head lice, which method comprises topically applying to said subject a first and a second pediculicide, said first pediculicide being a carbamate or organophosphate pediculicide and said second pediculicide being a pyrethroid or pyrethrin pediculicide, characterized in that said second pediculicide is applied between 15 minutes and 12 hours after the application of said first pediculicide.

Viewed from a still further aspect the invention provides the use of a carbamate or organophosphate pediculicide and a pyrethroid or pyrethrin pediculicide for the preparation of topical pediculicide compositions for time-staggered topical application to a human subject to combat infestation by multicellular ectoparasites with exoskeletons.

The time period between application of the two pediculicides is preferably 20 minutes to 4 hours, more preferably 30 minutes to 3 hours, especially about 2 hours.

Treatment of head lice according to the invention, which may be to kill lice present in the hair or to kill any lice which it is thought might be present in the hair, is preferably combined with combing of the hair with a fine-toothed comb, eg a "nit comb". Such combs have long been widely available. Combing may be effected before, during or after the method of the invention, preferably after, and preferably repeatedly. Combing is best effected when the hair is wet and particularly when the hair has been treated with a conditioner.

The two pediculicide compositions take any convenient topical application form selected from solution, cream, gel, cream rinse, dispersion, powder, lotion, spray, or unguent. However, at least one of the compositions is a shampoo, ie a surfactant containing composition, or cream rinse. It is particularly preferred that the later applied composition is a shampoo or cream rinse.

In an especially preferred embodiment, the first-applied composition is an organophosphate-containing gel or solution, eg one containing an alcohol such as isopropanol, or a physiologically tolerable carbamate formulation, eg a lotion, and the later applied composition is a pyrethroid-containing shampoo or cream rinse. Permethrin shampoos are frequently actually cream rinses (conditioners).

The organophosphate pediculicide used according to the invention may be any organophosphate with ectoparasite killing effect which is physiologically tolerable on dermal application. Examples of such compounds include malathion, parathion, dichlorvos, chlorpyrifos, chlorthion, trichlorphon, methyl parathion, and fenchlorphos. The use of malathion however is preferred. Where a carbamate pediculicide is used, this may be any carbamate with ectoparasite killing effect which is physiologically tolerable on dermal application. One examples of such a compound is carbaryl. The use of an organophosphate however is preferred.

For treatment of head lice in particular, the organophosphate or carbamate is preferably present in the pediculicide composition at a concentration of 0.02 to 0.4% wt, especially 0.04 to 0.2% wt, particularly about 0.1% wt. The remaining components of the composition may be conventional components for topical compositions and may be present in conventional amounts, eg water, alcohols, gelling agents, surfactants, fragrances, etc.

The pyrethroid or pyrethrin pediculicide used according to the invention may be any pyrethroid or pyrethrin with ectoparasite killing effect which is physiologically tolerable on dermal application. Examples of such pyrethroid compounds, which are generally preferred relative to the pyrethrins, include permethrin, phenothrin, cypermethrin, pyrethrin and deltamethrin. The use of permethrin however is preferred. The pyrethrins, if used, may for example be derived from natural sources such as the chrysanthemum plant. However, where pyrethrins are used, it is preferred also to use a synergist (as discussed below).

For treatment of head lice in particular, the pyrethroid or pyrethrin is preferably present in the pediculicide composition at a concentration of 0.2 to 3% wt, especially 0.5 to 2% wt, particularly about 1% wt. The remaining components of the composition may be conventional components for topical compositions and may be present in conventional amounts, e.g. water, alcohol, gelling agents, surfactants, fragrances, etc.

For treatment of other ectoparasites, the pediculicide contents of the compositions may be adjusted appropriately. Thus, for example, for treatment of scabies (where the compositions will generally be applied in cream, gel or lotion form, especially cream form), the pediculicide contents may be up to five times the preferred contents recited above for head lice.

It is especially preferred that one or both of the pediculicide compositions, especially the pyrethroid or pyrethrin composition, should contain a monooxygenase inhibitor as a synergist for the pyrethroid/pyrethrin, eg piperonyl butoxide. It is also preferred that one or both of the pediculicide compositions should contain an abrasive, eg silicate or diatoms, to assist in disrupting the exoskeleton of the ectoparasite.

In the invention, the pediculicides may be applied to any surface of the body, especially hair bearing surfaces, and preferably the head. Application may be preceded by, accompanied by or followed by washing and/or rinsing. Particularly preferably, the application of the later composition is followed by rinsing. Desirably the organophosphate or carbamate composition is left in contact with the hair for 15 minutes to 12 hours, especially 20 minutes to 4 hours, more preferably 30 minutes to 3 hours, most preferably about 2 hours. The pyrethroid composition is preferably left in contact with the skin and hair for 5 minutes to several hours, eg 15 to 30 minutes, depending on the nature of the formulation used. A cream formulation would typically be left in contact with the skin and hair for up to 12 hours before washing, while a shampoo would typically be used by washing the hair for about 10 minutes followed by rinsing with water. The method of the invention may if necessary be repeated, eg after 7 to 10 days, but for a single case of infestation a single performance of the method will generally be sufficient.

The organophosphate or carbamate compositions used in the invention generally contain lower concentrations of the organophosphate or carbamate component than is conventional for treatment for head lice and form a further aspect of the invention. Thus viewed from this further aspect the invention provides a topical pediculicide composition comprising a physiologically tolerable carrier and from 0.02 to 0.4% wt, preferably 0.04 to 0.2% wt, especially about 0.1% wt, of an organophosphate or carbamate pediculicide, preferably a composition in gel, cream, shampoo or solution form.

Compositions containing malathion and an alcohol, eg isopropanol, are especially preferred.

One or both of the pediculicide compositions may advantageously contain a further pediculicide, e.g. selected from the chloronicotinyl (eg imidacloprid), phenylpyrazole (eg fipronil), oxadiazine (eg indoxacarb), pyrazole (eg chlorfenapyr), or organochlorine (eg lindane) classes.

The pediculicide compositions are preferably packaged in plastic tubes, single-use sachets, or glass vials.

The invention may also be used in the treatment of other ectoparasites, eg fleas, ticks, other lice (eg *Pediculus humanus humanus* and *Phtirus pubis*) and mites. Scabies which results from infestation by the mite *Sarcoptes scabiei,* is one condition in particular that can be treated.

Where the ectoparasite infestation, eg scabies, is associated with hair-free areas of the skin, topical application will be to the affected area and optionally to areas deemed to be at risk of infestation. This of course applies to all treatments according to the invention.

The invention will now be described further with reference to the following non-limiting Examples.

### Example 1

### Organophosphate Gel composition

Malathion in concentrated solution in isopropanol is added at 0.1% wt to a commercially available hair gel, e.g. Essentials Hair Gel from The Boots Company plc.

### Example 2

### Pyrethroid Shampoo Composition

Permethrin is added at 1% wt to a commercially available shampoo, e.g. Head & Shoulders from Proctor & Gamble, or cream rinse/conditioner.

### Example 3

### Head lice treatment kit

A kit is prepared comprising a paper box, a vial containing 30 mL of the composition of Example 1, a vial containing 30 mL of the composition of Example 2, and an insert carrying instructions for use.

### Example 4

### Scalp Treatment

About 25 ml of the gel of Example 1 is massaged into the hair and scalp of a lice-infested schoolchild. After 30 minutes the hair is rinsed. After a further ninety minutes the hair is wetted and about 25 ml of the shampoo of Example 2 is rubbed into the hair to create a lather. The hair is rinsed after 10 minutes. The following day the hair is combed wet with a nit comb.

### Example 5

### Trial

A 10 year old girl had for over 4 months used a 0.5% solution of malathion (Prioderm (Trade Mark) lotion from Mundipharma) to combat head lice without any significant clinical effect. This subject then used a 1% malathion shampoo (Prioderm (Trade Mark) shampoo from Mundipharma) for 30 minutes, washed her hair with water, then used a 1% permethrin shampoo (Nix (Trade Mark) shampoo (actually a cream rinse rather than strictly speaking a shampoo)from ACO HUD) for 10 minutes. This treatment was repeated after 7 days and head lice infestation was cured.

## Claims

1. A pyrethroid or pyrethrin pediculicide for use in a method of treatment of a human subject to combat infestation by multicellular ectoparasites with exoskeletons, which method comprises topically applying to said subject a first and a second pediculicide, said first pediculicide being a carbamate or organophosphate pediculicide and said second pediculicide being a pyrethroid or pyrethrin pediculicide, **characterized in that** said second pediculicide is applied between 15 minutes and 12 hours after the application of said first pediculicide, and wherein the two pediculicides are applied as a composition for topical application in the form of a solution, a cream, a gel, a cream rinse, a dispersion, a powder, a lotion, a spray or an unguent, with at least one of the compositions being a shampoo or a cream rinse.

2. An organophosphate or carbamate pediculicide for use in a method of treatment of a human subject to combat infestation by multicellular ectoparasites with exoskeletons, which method comprises topically applying to said subject a first and a second pediculicide, said first pediculicide being a carbamate or organophosphate pediculicide and said second pediculicide being a pyrethroid or pyrethrin pediculicide, **characterized in that** said second pediculicide is applied between 15 minutes and 12 hours after the application of said first pediculicide, and wherein the two pediculicides are applied as a composition for topical application in the form of a solution, a cream, a gel, a cream rinse, a dispersion, a powder, a lotion, a spray or an unguent, with at least one of the compositions being a shampoo or a cream rinse.

3. The pediculicide as claimed in claim 1 or claim 2 wherein the ectoparasites are head lice.

4. The pediculicide as claimed in any one of the preceding claims wherein said second pediculicide is applied 20 minutes to 4 hours after the application of said first pediculicide.

5. The pediculicide as claimed in any one of the preceding claims wherein said second pediculicide is applied 30 minutes to 3 hours after the application of said first pediculicide.

6. The pediculicide as claimed in claim 3 wherein the organophosphate or carbamate is present in a first pediculicide composition at a concentration of 0.02 to 0.4% wt..

7. The pediculicide as claimed in claim 3 wherein the pyrethroid or pyrethrin is present in a second pediculicide composition at a concentration of 0.2 to 3% wt..

8. The pediculicide as claimed in any one of the preceding claims wherein the first pediculicide is applied as an organophosphate-containing gel or solution or a physiologically tolerable carbamate formulation and the second pediculicide is applied as a pyrethroid-containing shampoo or cream rinse.

9. The pediculicide as claimed in any one of the preceding claims wherein the first pediculicide is an organophosphate.

10. The pediculicide as claimed in any one of the preceding claims wherein the first pediculicide is malathion and the second pediculicide is permethrin.

11. The use of a pyrethroid or pyrethrin pediculicide and an organophosphate or carbamate pediculicide for the preparation of topical pediculicide compositions for use in a method of treatment of a human subject to combat infestation by multicellular ectoparasites with exoskeletons, which method comprises topically applying to said subject a first and a second pediculicide, said first pediculicide being a carbamate or organophosphate pediculicide and said second pediculicide being a pyrethroid or pyrethrin pediculicide, **characterized in that** said second pediculicide is applied between 15 minutes and 12 hours after the application of said first pediculicide, and wherein the two pediculicides are applied as a composition for topical application in the form of a solution, a cream, a gel, a cream rinse, a dispersion, a powder, a lotion, a spray or an unguent, with at least one of the compositions being a shampoo or a cream rinse.

## Patentansprüche

1. Pyrethroid- oder Pyrethrin-Pediculizid zur Verwendung bei einem Verfahren zur Behandlung eines menschlichen Patienten zur Bekämpfung von Befall durch mehrzellige Ektoparasiten mit Exoskeletten, wobei das Verfahren Folgendes umfasst: topische Verabreichung eines ersten und eines zweiten Pediculizids an den Patienten, wobei es sich bei dem ersten Pediculizid um ein Carbamat- oder Organophosphat-Pediculizid und bei dem zweiten Pediculizid um ein Pyrethroid- oder Pyrethrin-Pediculizid handelt, **dadurch gekennzeichnet, dass** das zweite Pediculizid zwischen 15 Minuten und 12 Stunden nach der Verabreichung des ersten Pediculizids verabreicht wird, und wobei die beiden Pediculizide als Zusammensetzung für die topische Verabreichung in Form einer Lösung, einer Creme, eines Gels, einer Cremespülung, einer Dispersion, eines Pulvers, einer Lotion, eines Sprays oder einer Salbe verabreicht werden, wobei es sich bei mindestens einer der Zusammensetzungen um ein Shampoo oder eine Cremespülung handelt.

2. organophosphat- oder Carbamat-Pediculizid zur Verwendung bei einem Verfahren zur Behandlung eines menschlichen Patienten zur Bekämpfung von Befall durch mehrzellige Ektoparasiten mit Exoskeletten, wobei das verfahren Folgendes umfasst: topische Verabreichung eines ersten und eines zweiten Pediculizids an den Patienten, wobei es sich bei dem ersten Pediculizid um ein Carbamat- oder organophosphat-Pediculizid und bei dem zweiten Pediculizid um ein Pyrethroid- oder Pyrethrin-Pediaulizid handelt, **dadurch gekennzeichnet, dass** das zweite Pediculizid zwischen 15 Minuten und 12 Stunden nach der Verabreichung des ersten Pediculizids verabreicht wird, und wobei die beiden Pediculizide als zusammensetzung für die topische Verabreichung in Form einer Lösung, einer Creme, eines Gels, einer Cremespülung, einer Dispersion, eines Pulvers, einer Lotion, eines Sprays oder einer Salbe verabreicht werden, wobei es sich bei mindestens einer der Zusammensetzungen um ein Shampoo oder eine Cremespülung handelt.

3. Pediculizid nach Anspruch 1 oder Anspruch 2, wobei es sich bei den Ektoparasiten um Kopfläuse handelt.

4. Pediculizid nach einem der vorhergehenden Ansprüche, wobei das zweite Pediculizid 20 Minuten bis 4 Stunden nach der Verabreichung des ersten Pediculizids verabreicht wird.

5. Pediculizid nach einem der vorhergehenden Ansprüche, wobei das zweite Pediculizid 30 Minuten bis 3 Stunden nach der Verabreichung des ersten Pediculizids verabreicht wird.

6. Pediculizid nach Anspruch 3, wobei das organophosphat oder Carbamat in einer ersten Pediculizidzusammensetzung in einer Konzentration von 0,02 bis 0,4 Gew.-% vorliegt.

7. Pediculizid nach Anspruch 3, wobei das Pyrethroid oder Pyrethrin in einer zweiten Pediculizidzusammensetzung in einer Konzentration von 0,2 bis 3 Gew.-% vorliegt.

8. Pediculizid nach einem der vorhergehenden Ansprüche, wobei das erste Pediculizid als organophosphathaltiges Gel oder als organophvsphathaltige Lösung oder als physiologisch annehmbare Carbamatformulierung und das zweite Pediculizid als pyrethroidhaltiges Shampoo oder als pyrethroidhaltige Cremespülung verabreicht wird.

9. Pediculizids nach einem der vorhergehenden Ansprüche, wobei es sich bei dem ersten Pediculizid um ein Organophosphat handelt.

10. Pediculizid nach einem der vorhergehenden Ansprüche, wobei es sich bei dem ersten Pediculizid um Malathion und bei dem zweiten Pediculizid um Permethrin handelt.

11. Verwendung eines Pyrethroid- oder Pyrethrin-Pediculizids und eines Organophosphat- oder Carbamat-Pediculizids für die Herstellung von topischen Pediculizidzusammensetzungen zur Verwendung bei einem Verfahren zur Behandlung eines menschlichen Patienten zur Bekämpfung von Befall durch mehrzellige Ektoparasiten mit Exoskeletten, wobei das Verfahren Folgendes umfasst: topische Verabreichung eines ersten und eines zweiten Pediculizids an den Patienten, wobei es sich bei dem ersten Pediculizid um ein Carbamat- oder Organophosphat-Pediculizid und bei dem zweiten Pediculizid um ein Pyrethroid- oder Pyrethrin-Pediculizid handelt, **dadurch gekennzeichnet, dass** das zweite Pediculizid zwischen 15 Minuten und 12 Stunden nach der Verabreichung des ersten Pediculizids verabreicht wird, und wobei die beiden Pediculizide als Zusammensetzung für die topische Verabreichung in Form einer Lösung, einer Creme, eines Gels, einer Cremespülung, einer Dispersion, eines Pulvers, einer Lotion, eines Sprays oder einer Salbe verabreicht werden, wobei es sich bei mindestens einer der Zusammensetzungen um ein Shampoo oder eine Cremespülung handelt.

## Revendications

1. Pédiculicide à base de pyréthrinoide ou de pyréthrine pour une utilisation dans une méthode de traitement d'un sujet humain afin de lutter contre une infestation par des ectoparasites multicellulaires ayant des exosquelettes, laquelle méthode comprend 7.'application topique audit sujet d'un premier et d'un deuxième pédiculicide, ledit premier pédiculicide étant un pédiculicide à base de carbamate ou d'un composé organophosphoré et ledit deuxième pédiculicide étant un pédiculicide à base de pyréthrinoide ou de pyréthrine, **caractérisé en ce que** ledit deuxième pédiculicide est appliqué entre 15 minutes et 12 heures après l'application dudit premier pédiculicide, et **caractérisé en ce que** les deux pédiculicides sont appliqués sous forme de composition pour une application topique sous la forme d'une solution, d'une crème, d'un gel, d'un après-shampooing, d'une dispersion, d'une poudre, d'une lotion, d'un spray ou d'un onguent, au moins l'une des compositions étant un shampooing ou un après-shampooing.

2. Pédiculicide à base de carbamate ou d'un composé organophosphoré pour une utilisation dans une méthode de traitement d'un sujet humain afin de lutter contre une infestation par des ectoparasites multicellulaires ayant des exosquelettes, laquelle méthode comprend l'application topique audit sujet d'un premier et d'un deuxième pédiculicide, ledit premier pédiculicide étant un pédiculicide à base de carbamate ou d'un composé organophosphoré et ledit deuxième pédiculicide étant un pédiculicide à base de pyréthrinoïde ou de pyréthrine, **caractérisé en ce que** ledit deuxième pédiculicide est appliqué entre 15 minutes et 12 heures après l'application dudit premier pédiculicide, et **caractérisé en ce que** les deux pédiculicides sont appliqués sous forme de composition pour une application topique sous la forme d'une solution, d'une crème, d'un gel, d'un après-shampooing, d'une dispersion, d'une poudre, d'une lotion, d'un spray ou d'un onguent, au moins l'une des compositions étant un shampooing ou un après-shampooing,

3. Pédiculicide selon la revendication 1 ou la revendication 2, **caractérisé en ce que** les ectoparasites sont des poux de tête.

4. Pédiculicide selon l'une quelconque des revendications précédentes, **caractérisé en ce que** ledit deuxième pédiculicide est appliqué de 20 minutes à 4 heures après l'application dudit premier pédiculicide.

5. Pédiculicide selon l'une quelconque des revendications précédentes, **caractérisé en ce que** ledit deuxième pédiculicide est appliqué de 30 minutes à 3 heures après l'application dudit premier pédiculicide.

6. Pédiculicide selon la revendication 3, **caractérisé en ce que** le carbamate ou le composé organophosphoré est présent dans une première composition de pédiculicide selon une concentration allant de 0,02 à 0,4% en poids.

7. Pédiculicide selon la revendication 3, **caractérisé en ce que** le pyréthrinoïde ou la pyréthrine est présent dans une deuxième composition de pédiculicide selon une concentration allant de 0,2 à 3% en poids.

8. Pédiculicide selon l'une quelconque des revendications précédentes, **caractérisé en ce que** ledit premier pédiculicide est appliqué sous forme de gel ou solution contenant un composé organophosphoré ou d'une formulation de carbamate physiologiquement tolérable, et le deuxième pédiculicide est appliqué sous forme de shampooing ou d'après-shampooing contenant du pyréthrinoïde.

9. Pédiculicide selon l'une quelconque des revendications précédentes, **caractérisé en ce que** le premier pédiculicide est un composé organophosphoré.

10. Pédiculicide selon l'une quelconque des revendications précédentes, **caractérisé en ce que** le premier pédiculicide est du malathion et le deuxième pédiculicide est de la perméthrine.

11. Utilisation d'un pédiculicide à base de pyréthrinoïde ou de pyréthrine et d'un pédiculicide à base de carbamate ou d'un composé organophosphoré, pour la préparation de compositions pédiculicides topiques pour une utilisation dans une méthode de traitement d'un sujet humain afin de lutter contre une infestation par des ectoparasites multicellulaires ayant des exosquelettes, laquelle méthode comprend l'application topique audit sujet d'un premier et d'un deuxième pédiculicide, ledit premier pédiculicide étant un pédiculicide à base de carbamate ou d'un composé organophosphoré et ledit deuxième pédiculicide étant un pédiculicide à base de pyréthrinoïde ou de pyréthrine, **caractérisé en ce que** ledit deuxième pédiculicide est appliqué entre 15 minutes et 12 heures après l'application dudit premier pédiculicide, et **caractérisé en ce que** les deux pédiculicides sont appliqués sous forme de composition pour une application topique sous la forme d'une solution, d'une crème, d'un gel, d'un après-shampooing, d'une dispersion, d'une poudre, d'une lotion, d'un spray ou d'un onguent, au moins l'une des compositions étant un shampooing ou un après-shampooing.
